(19) Europäisches Patentamt
European Patent Office
Office européen des brevets

(11) Publication number: **0 547 705 A1**

# (12) EUROPEAN PATENT APPLICATION

(21) Application number: **92203913.6**

(22) Date of filing: **15.12.92**

(51) Int. Cl.5: **C07D 513/04**, A61K 31/55, //(C07D513/04,281:00,231:00), (C07D513/04,333:00,281:00), (C07D513/04,281:00,277:00)

(30) Priority: **18.12.91 ES 9102818**

(43) Date of publication of application: **23.06.93 Bulletin 93/25**

(84) Designated Contracting States: **AT BE CH DE DK ES FR GB GR IE IT LI LU MC NL PT SE**

(71) Applicant: **CONSEJO SUPERIOR DE INVESTIGACIONES CIENTIFICAS**
**Serrano, 117**
**E-28006 Madrid(ES)**
Applicant: **ADIR ET COMPAGNIE**
**1 rue Carle Hébert**
**F-92415 Courbevoie Cédex(FR)**

(72) Inventor: **Vega Noverola, Salvador**
**Plaza de los Reyes Magos, 12-11o**
**E-28007 Madrid(ES)**
Inventor: **Diaz Martin, Juan Antonio**
**Anastro, 23-1o**
**E-28033 Madrid(ES)**

(74) Representative: **Ungria Lopez, Javier et al**
**Avda. Ramon y Cajal, 78**
**E-28043 Madrid (ES)**

## (54) Pharmacologically active tricyclic benzotriazepine derivatives.

(57) The present invention refers to a new group of chemical substances of general formula I, and intermediates for the preparation thereof, which are useful, among other uses, as psychotropic agents. It also refers to the processes used to obtain them. The new compounds of this invention include 5,10-dihydro-4,4-dioxothieno[3,2-c][2,1]-benzothiazepine;5,10-dihydro-4,4-dioxothieno[3,4-c][2,1]-benzothiazepine; 4,9-dihydro-10,10-dioxothieno-[2,3-c][2,1]-benzothiazepine; 4,9-dihydro-10,10-dioxo-2H-pyrazolo[3,4-c][2,1]benzothiazepine,4,9-dihydro-10,10-dioxo-1H-pyrazolo[3,4-c][2,1]benzothiazepine and 4,9-dihydro-10,10-dioxothiazolo[5,4-c][2,1]benzothiazepine systems, which are new heterocyclic systems, described for the first time in the present invention. The derivatives of formula I are basic compounds, due to the presence of an aminic nitrogen and they form stable salts with several acids. They are useful as antidepressant, diuretic, antihypertensive, antihistaminic, anticonvulsive, antipyretic, anti-inflammatory and/or analgesic agents.

EP 0 547 705 A1

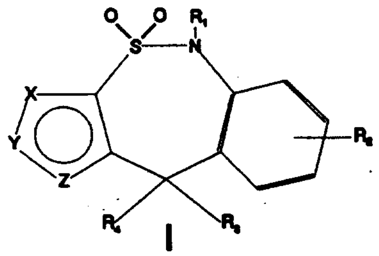

I

Technical Field

The present invention refers to a new group of chemical substances of general formula I, and intermediates for the preparation thereof, which are useful, among other uses, as psychotropic agents.

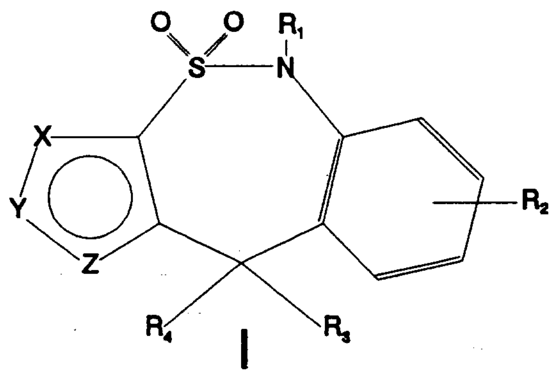

I

Prior Art

There are described in the bibliography numerous derivative compounds of the tricyclic system.

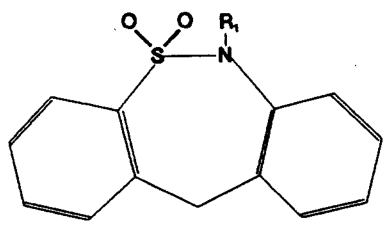

and the only teaching concerning the substitution of a benzene ring by a hetaryl ring is found in patent ES 9001576 that describes the synthesis of trioxo-thieno[2,-1]benzothiazepine derivatives:

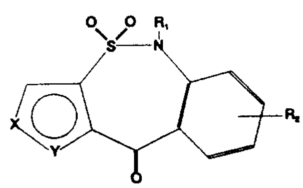

which noticeably differ from the ones corresponding to the present invention, among other differences, because none of them include the ketone group.

Description of the invention

The new compounds of this invention include 5,10-dihydro-4,4-dioxothieno[3,2-c][2,1]benzothiazepine; 5,10-dihydro-4,4-dioxothieno[3,4-c][2,1]benzothiazepine; 4,9-dihydro-10,10-dioxothieno [2,3-c][2,1]-benzothiazepine; 4,9-dihydro-10,10-dioxo-2H-pyrazolo[3,4-c][2,1]benzothiazepine, 4,9-dihydro-10,10-dioxo-1H-pyrazolo [3,4-c] 2-,1]benzothiazepine and 4,9-dihydro-10,10-dioxothiazolo[5,4-c][2,1]benzothiazepine systems, which are new heterocyclic systems, described for the first time by the present inventors. The representative systems of said structures are shown in formula II.

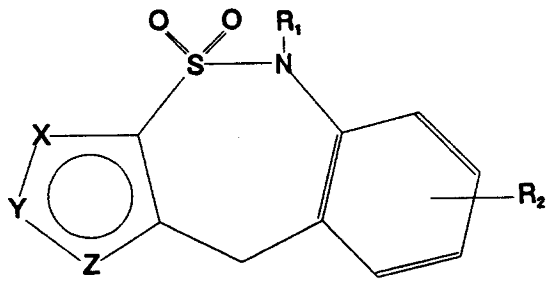

II

The pharmacologically active compounds of the present invention correspond to derivatives of general formula I, wherein X represents a nitrogen, sulfur atom or a NH, N-alkyl, N-arylalkyl or CH group; Y represents a nitrogen, sulfur atom or a NH, N-alkyl, N-arylalkyl, CH or C-alkyl group and Z is a nitrogen, sulfur atom or a CH, C-alkyl or C-aryl group, it being understood that the term alkyl means a linear or branched group of 1 to 6 carbon atoms.

$R_1$ represents a hydrogen atom, linear or branched alkyl groups or arylalkyl groups in which there may be functional substitutions in their nucleus or branches in their chain. $R_2$ indicates one, or several, substituents bonded to the benzene portion of the structure, chosen among hydrogen, halogen atoms and/or nitro, amino, lower-alkyl amino, lower-dialkyl amino, cyano, sulfonamido, trifluoromethyl and alkyl groups or alkoxyl with 1 to 6 carbon atoms.

$R_3$ and $R_4$, identical or different, represent independently one from another, a hydrogen, halogen atom, a hydroxy, lower-alkoxy, mercapto, lower alkylthio, amino, lower-alkyl amino, lower-dialkyl amino atom or a group of formula III.

T-(Alk)$_n$-Het III

wherein

- T represents an oxygen, nitrogen or sulfur atom
- n equals 0 or 1
- Alk represents a linear or branched alkyl chain of 1 to 5 carbon atoms
- Het represents a mono or bicyclic saturated heterocyclic system, with 5 to 7 vertexes in each cycle optionally including 1 or 2 heteroatoms chosen among nitrogen, oxygen and sulfur with the condition that the heterocyclic system contains at least one nitrogen atom, each nitrogen atom being optionally substituted by a lower-alkyl, phenyl, phenyl lower-alkyl group, the phenyl nucleus also being able to be substituted by one or several, halogen atoms or lower-alkyl, lower-alkoxy or trifluoromethyl groups. Het may also be represented by a group of formula

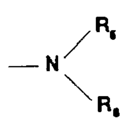

wherein $R_5$ and $R_6$, identical or different, represent independently one from another, a hydrogen atom, a lower-alkyl or lower-arylalkyl group $R_3$ and $R_4$ can bond together forming an imino, hydroxyimino, lower-alkoxy imino group or a group of formula

= N-(Alk)$_n$-Het IVa

or a group of formula

= N-O-(Alk)$_n$-Het IVb

or a group of formula

= (Alk)$_n$-Het IVc

or a group of formula

= Het IVd

wherein Het, Alk and n correspond to the above given meaning. It is understood that lower-alkyl, lower-alkoxy and lower alkylthio groups are linear or branched groups containing 1 to 6 carbon atoms.

The compounds of formula I may be in the form of isomers, diastereoisomers or enantiomers, together or separate.

The compounds of formula I are prepared from ketones of formula V, whose symbols X, Y, Z, $R_1$ and $R_2$ have the same meanings as those described above.

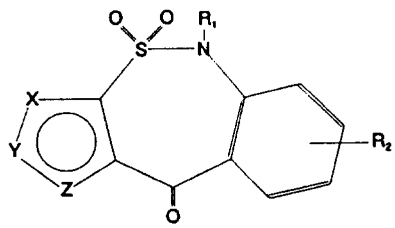

V

In turn, these ketones are obtained by methods known in the prior art, using chlorosulfonyl derivatives of formula VI as starting products (J. Org. Chem. 45, 617 (1980), Org. Prep. Proc. Int. 17, 163 (1985); J. Heterocyclic Chem. 21, 1017 (1984)).

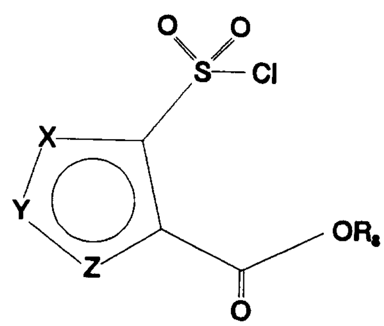

VI

$R_8$ means a linear or branched alkyl group of 1 to 4 carbon atoms and X, Y, Z have the same meanings as in the above cases.

When $R_3$ is hydrogen and $R_4$ is aminoalkyloxy, aminoalkylthio or aminoalkylamino, the compounds of formula I are prepared by alkylation of the corresponding alcohols, thiols or tricyclic amines, obtained from the ketones of formula V by usual methods, according to the following illustrative diagram:

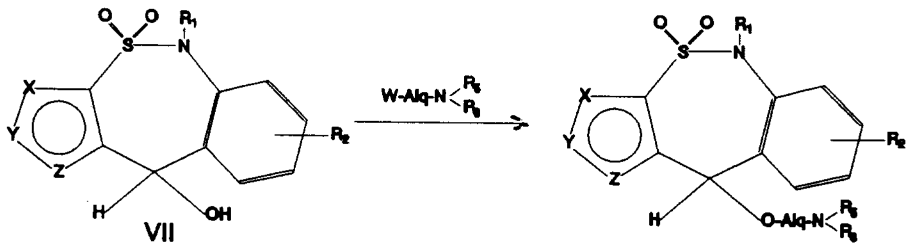

VII

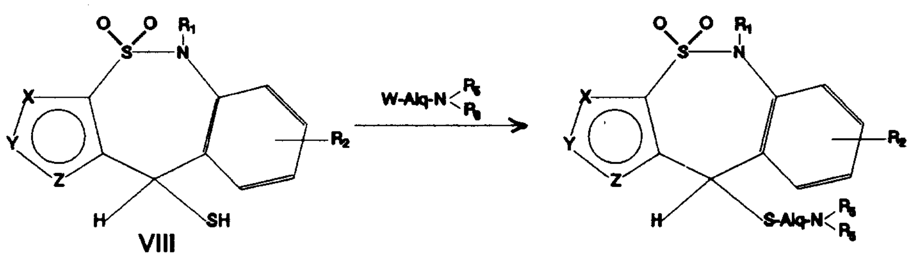

VIII

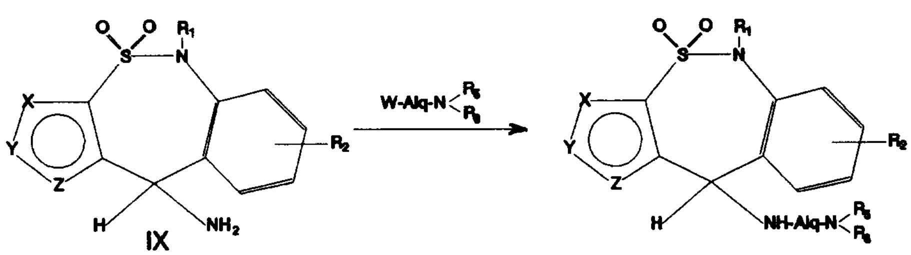

IX

These compounds are also prepared by alkylation of hydroxyalkylamines, mercaptoalkylamines and aminoalkylamines, with examples of these tricyclic derivatives that have a W reactive group (Formula X), obtained from alcohols of formula VII, according to the following reaction diagram:

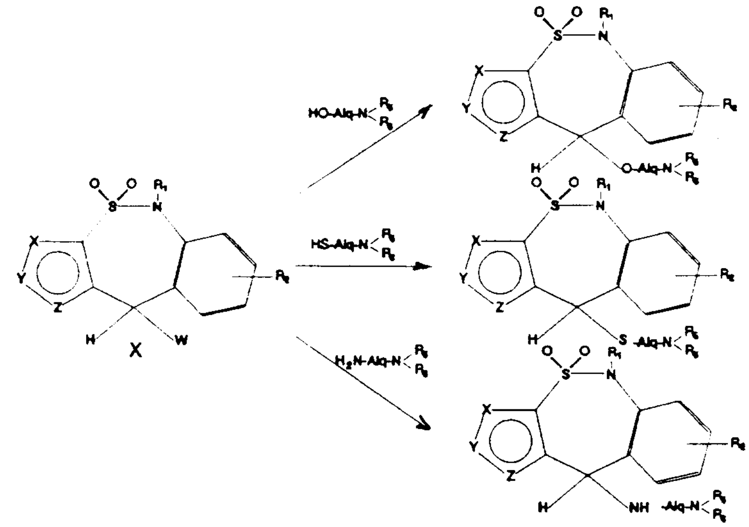


When, in Formula I, $R_3$ is hydrogen and $R_4$ is an aminoalkyloxy group, these compounds are alternatively formed by dehydration of the tricyclic alcohol ($R_3$ = hydrogen; $R_4$ = hydroxyl) and a hydroxyalkylamine, or else by dehydration with an alcohol having a W reactive group, separated by, at least, two carbon atoms of the hydroxyl group, in order to form a derivative of formula XI and subsequent reaction of the latter with amines, according to the following illustrative diagram. In all of these derivatives of formula VII to X, X, $R_1$, $R_2$, X, Y, Z have the meaning as that of formula I.

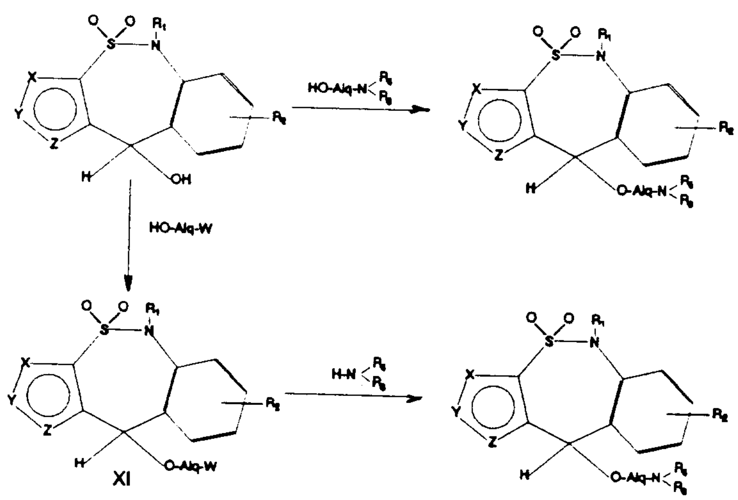


In these diagrams the W reactive group represents chlorine, bromine, iodine, alkylsulfate, alkylsulfonate, arylsulfonate or alkylphosphate, and the rest of the symbols have meanings similar to those explained above.

By alkylation of the oximes of formula XII the desired aminoalkoxyiminic derivatives are synthesized. Said alkylation is carried out by reaction between an oxime salt, preferably of alkaline metal, and a conveniently substituted aminoalkyl derivative, as is represented in the following diagram.

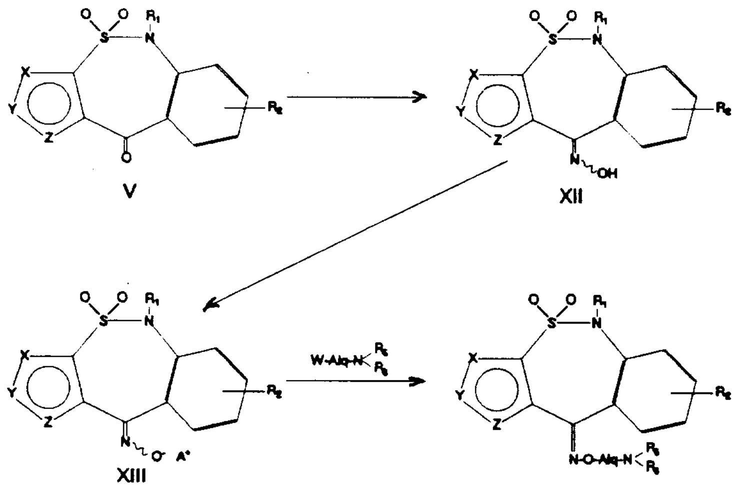


V XII XIII

In this diagram the meanings of all of the symbols are identical to those explained above, and either implies treatment of the oxime in an anhydrous medium with a strong base, such as an alkaline metal alkoxide, preferably sodium ethoxide, an alkaline metal, or alkaline-earth hydride, or an alkyl lithium, or else treatment of the oxime in a heterogenous basic medium and in the presence of a phase transfer catalyst, preferably an ammonium, phosphonium salt or a crown ether.

This type of compounds of formula I ($R_3R_4$ = aminoalkoxyimino) can be alternatively synthesized by reaction between a hydroxyalkylamine and a derivative of formula XIV.

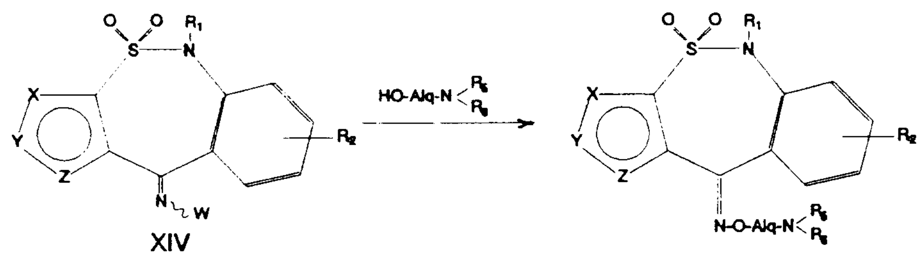


XIV

In which case the W reactive group has the meaning of an alkylsulfonate, arylsulfonate or alkylphosphate, and the rest of the symbols are similar to those already described.

The third possible way to form this type of tricyclic aminoalkoxyimino derivatives consists of direct condensation of the ketones of formula V with conveniently substituted hydroxylamines, as is illustrated in the following diagram:

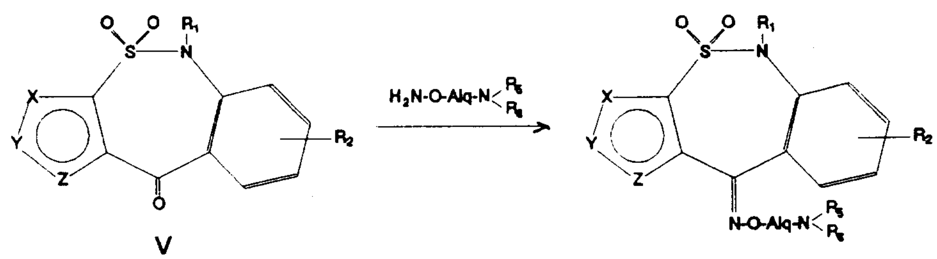


V

The symbols X, Y, Z, $R_1$, $R_2$, Alk, $R_5$, $R_6$, $R_3$ and $R_4$ have the already known meanings.

Finally, the compounds of formula I, wherein $R_3R_4$ have the meaning of an aminoalkylidenic grouping, are preferably prepared by reaction of the ketones of formula V with a Grignard reagent of an aminoalkyl

halide or a nitrogenated heterocycle halide. Chlorides, bromides and iodides are useful as halides. Such Grignard reagents are prepared and reacted in the normal conditions for this type of reactions, for example, formation of the reagent is carried out by reaction between the halide and magnesium turnings, catalyzed by an iodine crystal or some drops of ethyl bromide, in an inert solvent, preferably anhydrous tetrahydrofuran. The synthesis of hydroxylic intermediates of formula I, represented in the diagram, is produced by contacting the organomagnesium solution with another ketone solution of formula V, in the same inert solvent and subsequent hydrolysis of the complex with an aqueous ammonium chloride solution.

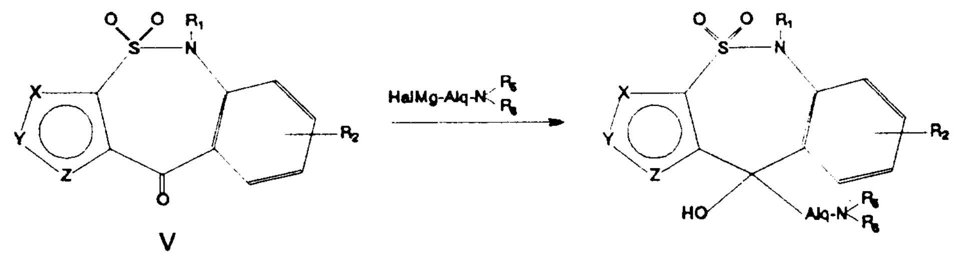

V

In the above diagram the symbol Hal means chlorine, bromine or iodine and the rest of the symbols have the same meanings as those given above.

The hydroxylic derivatives of formula I are dehydrated in acid conditions, to yield the aminoalkylidenes of formula I. Said dehydration is carried out by treatment of the hydroxylic derivatives with strong acids, such as hydrochloric acid, hydrobromic acid, hydroiodic acid, sulfuric acid and organic sulfonic acids, or else with organic dehydrating agents such as acetic anhydride, or inorganic dehydrating agents that include phosphorous oxychloride, thionyl chloride, sulfuryl chloride, phosphorus pentachloride, etc.

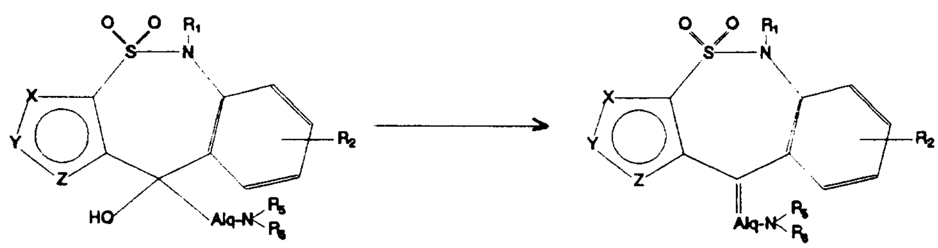

I I

Wherein the derivatives of formula I have meanings similar to those described above in this invention.

Other ways to obtain tricyclic aminoalkylidene derivatives, well documentated in the prior art, such as the use of ylides, are also possible as to their use in the synthesis of this type of compound of formula I.

Given that the synthesis of all the compounds described by formula I can give rise to the formation of isomers, enantiomers or diastereoisomers, as it has been said above, a subsequent step of separation of said isomers by normal processes may be necessary. Despite this, the formation of isomers does not invalidate the method as a method of preparation.

Examples

Examples 1-52 illustrate the preparation of intermediate compounds, which are likewise part of the invention in the same way as the pharmacologically active compounds. Examples 53-277 illustrate the preparation of the pharmacologically active compounds of formula I.

Example 1

3.78 g. (0.1mol) of sodium boron hydride are added in portions to a suspension of 13.96 g. (0.05 mol ) of 5,10-dihydro-5-methyl-4,4,10-trioxoethieno[3,2-c][2,1]-benzothiazepine in 100 ml. of methanol. Once all

the sodium boron hydride has been added (15 minutes), the mixture is stirred at room temperature for 1 more hour; the methanol is concentrated up to half its original volume and 100 ml. of ice water is added to it. In this way the 5,10-dihydro-4,4-dioxo-10-hydroxy-5-methylthieno [3,2-c][2,1]benzothiazepine is precipitated as a white solid with a melting point of 150-153 ºC (d) (toluene). I.R. (KBr): 3450 (OH); 1310. 1140 ($SO_2$).

Examples 2-18

The following derivatives are prepared by a process similar to that of example 1 and substitution of the X, Y, Z, $R_3$ and $R_4$ groups in formula I:

-5,10-Dihydro-4,4-dioxo-10-hydroxy-5-methylthieno[3,4-c][2,1]benzothiazepine (example 2.)
-4,9-Dihydro-10,10-dioxo-4-hydroxy-9-methylthieno[2,3-c][2,1]benzothiazepine (example 3.)
-4,9-Dihydro-10,10-dioxo-4-hydroxy-9-methyl1H-pyrazolo[3,4-c][2,1]benzothiazepine (example 4.)
-4,9-Dihydro-1,9-dimethyl-10,10-dioxo-4-hydroxy-1H-pyrazolo[3,4-c][2,1]benzothiazepine (example 5.)
-4,9-Dihydro-10,10-dioxo-1-ethyl-4-hydroxy-9-methyl-1H-pyrazolo[3,4-c][2,1]benzothiazepine (example 6.)
-1-Benzyl-4,9-dihydro-10,10-dioxo-4-hydroxy-9-methyl-1H-pyrazolo[3,4-c][2,1]benzothiazepine (example 7.)
-4,9-Dihydro-10,10-dioxo-4-hydroxy-1,3,9-trimethyl-1H-pyrazolo[3,4-c][2,1]benzothiazepine (example 8.)
-4,9-Dihydro-2,9-dimethyl-10,10-dioxo-4-hydroxy-2H-pyrazolo[3,4-c][2,1]benzothiazepine (example 9.)
-4,9-Dihydro-10,10-dioxo-2-ethyl-4-hydroxy-9-methyl-2H-pyrazolo[3,4-c][2,1]benzothiazepine (example 10.)
-2-Benzyl-4,9-dihydro-10,10-dioxo-4-hydroxy-9-methyl-2H-pyrazolo[3,4-c][2,1]benzothiazepine (example 11.)
-4,9-Dihydro-2,9-dimethyl-10,10-dioxo-4-hydroxythiazolo[5,4-c][2,1]benzothiazepine (example 12.)
-5,10-Dihydro-4,4-dioxo-10-mercapto-5-methylthieno[3,2-c][2,1] benzothiazepine (example 13.)
-5,10-Dihydro-4,4-dioxo-10-mercapto-5-methylthieno[3,4-c][2,1]benzothiazepine (example 14.)
-4,9-Dihydro-1,9-dimethyl-10,10-dioxo-4-mercapto-1H-pyrazolo[3,4-c][2,1]benzothiazepine (example 15.)
-4,9-Dihydro-2,9-dimethyl-10,10-dioxo-4-mercapto-2H-pyrazolo[3,4-c][2,1]benzothiazepine (example 16.)
-4,9-Dihydro-10,10-dioxo-2-ethyl-4-mercapto-9-methyl-2H-pyrazolo[3,4-c][2,1]benzothiazepine (example 17.)
-2-Benzyl-4,9-dihydro-10,10-dioxo-4-mercapto-9-methyl-2H-pyrazolo[3,4-c][2,1]benzothiazepine (example 18.)

Example 19

A mixture of 10.52 g. (0.04 mol ) of 4.9-dihydro-9-methyl-4,10,10-trioxo-1H-pyrazolo[3,4-c][2,1]-benzothiazepine, 11.12 g. (0.16 mol ) of hydroxylamine and 50 ml. of dry pyridine are refluxed for 24 hours. Once this period of time has gone by, and once the mixture is cold, the solution is poured on to 500 ml. of ice-water. It is stirred until the desired product crystallizes and it is filtered, washed with water and dried, yielding 4,9-dihydro-10,10-dioxo-4-hydroxyimino-9-methyl-1H-pyrazolo[3,4-c][2,1]benzothiazepine, as a white solid that turns out to be a mixture of both isomers Z and E.

Resolution of the isomers

(Z)-4,9-dihydro-10,10-dioxo-4-hydroxyimino-9-methyl-1H-pyrazolo[3,4-c][2,1]benzothiazepine

The previous crude mixture (2 g.) is dissolved in 50 ml. of boiling ethyl acetate and then cyclohexane is dropped upon this hot solution until a permanent turbidity appears. It is left to cool and the resulting crystals are filtered and dried, yielding the compound of the title as a white solid with a melting point of 243-246 ºC (d).

I.R. (KBr): 3150 (OH); 1350, 1150 ($SO_2$).

(E)-4,9-dihydro-10,10-dioxo-4-hydroxyimino-9-methyl-1H-pyrazolo[3,4-c][2,1]benzothiazepine

The mother liquors resulting from recrystallisation of the Z isomer are concentrated to dryness and the residue is chromatographed in a silica gel column , using a chloroform/ethanol mixture (9:1) as an eluent. Thus, by concentration of the suitable fractions the expected isomer is obtained as a white solid with a melting point of 239-242 $^{0}$C (d).

I.R. (KBr): 3250 (OH); 1350, 1150 ($SO_2$).

Examples 20-30

By repetition of the process of example 19 and the use of suitable tricyclic ketones the following hydroxyimino derivatives are prepared:

-5,10-Dihydro-4,4-dioxo-10-hydroxyimino-5-methylthieno[3,2-c][2,1] benzothiazepine (example 20.)

-5,10-Dihydro-4,4-dioxo-10-hydroxyimino-5-methylthieno[3,4-c][2,1]benzothiazepine (example 21.)

-4,9-Dihydro-10,10-dioxo-4-hydroxyimino-9-methylthieno[2,3-c][2,1]benzothiazepine (example 22.)

-4,9-Dihydro-1,9-dimethyl-10,10-dioxo-4-hydroxyimino-1H-pyrazolo[3,4-c][2,1]benzothiazepine (example 23.)

-4,9-Dihydro-10,10-dioxo-1-ethyl-4-hydroxyimino-9-methyl-1H-pyrazolo[3,4-c][2,1]benzothiazepine (example 24.)

-1-Benzyl-4,9-dihydro-10,10-dioxo-4-hydroxyimino-9-methyl-1H-pyrazolo[3,4-c][2,1]benzothiazepine (example 25.)

-4,9-Dihydro-10,10-dioxo-4-hydroxyimino-1,3,9-trimethyl-1H-pyrazolo[3,4-c][2,1]benzothiazepine (example 26.)

-4,9-Dihydro-2,9-dimethyl-10,10-dioxo-4-hydroxyimino-2H-pyrazolo[3,4-c][2,1]benzothiazepine (example 27.)

-4,9-Dihydro-10,10-dioxo-2-ethyl-4-hydroxyimino-9-methyl-2H-pyrazolo[3,4-c][2,1]benzothiazepine (example 28.)

-2-Benzyl-4,9-dihydro-10,10-dioxo-4-hydroxyimino-9-methyl-2H-pyrazolo[3,4-c][2,1]benzothiazepine (example 29.)

-4,9-Dihydro-2,9-dimethyl-10,10-dioxo-4-hydroxyiminothiazolo[5,4-c][2,1]benzothiazepine (example 30.)

Example 31

A solution of 14.05 g. (0.05 mol ) of the alcohol obtained in example 1, 3.54 ml. of 2-bromoethanol and 0.5 g. of hydrated p-toluenosulfonic acid in 300 ml of toluene is refluxed for 1 hour. In the course of the reaction the water formed is eliminated azeotropically, with the help of a Dean-Stark apparatus. Once the reaction has finished, the mixture is cooled and successively washed with 200 ml. of water, 100 ml. of 10% sodium bicarbonate solution and another 100 ml. of water. The toluene is dried over anhydrous sodium sulfate and concentrated at a reduced pressure obtaining 2-[5,10-dihydro-4,4-dioxo-5-methylthieno[3,2-c]-[2,1]benzothiazepin-10-yl)oxy]-1-bromoethane, as a white solid that is used in the subsequent reactions without further purification.

I.R. (KBr): 1315, 1140 ($SO_2$.)

Example 32

By a process similar to that of example 31, but replacing the bromoethanol by 3.35 ml. (0.05 mol ) of 2-chloroethanol, 2-[5,10-dihydro-4,4-dioxo-5-methylthieno[3,2-c][2,1]benzothiazepin-10-yl)oxy]-1-chloroethane is obtained as a white solid with a melting point of 111-113 ºC (of carbon tetrachloride.)

I.R. (KBr): 1320, 1140 ($SO_2$.)

Examples 33-52

In a way similar to that described in examples 31 and 32, the bromoethanes and chloroethanes of the following heterocyclic systems are obtained:

-2-[4,9-Dihydro-4,4-dioxo-5-methylthieno[3,4-c][2,1]benzothiazepin-10-yl)oxy] (examples 33 and 34.)

-2-[4,9-Dihydro-10,10-dioxo-9-methylthieno[2,3-c][2,1]benzothiazepin-4-yl)oxy] (examples 35 and 36.)

-2-[4,9-Dihydro-1,9-dimethyl-10,10-dioxo-1H-pyrazolo[3,4-c][2,1]benzothiazepin-4-yl)oxy] (examples 37 and 38.)

-2-[4,9-Dihydro-10,10-dioxo-1-ethyl-9-methyl-1H-pyrazolo[3,4-c][2,1]benzothiazepin-4-yl)oxy] (examples 39 and 40.)

-2-[1-Benzyl-4,9-dihydro-10,10-dioxo-9-methyl-1H-pyrazolo[3,4-c][2,1]benzothiazepin-4-yl)oxy] (examples 41 and 42.)

-2-[4,9-Dihydro-10,10-dioxo-1,3,9-trimethyl-1H-pyrazolo[3,4-c][2,1]benzothiazepin-4-yl)oxy] (examples 43 and 44.)

-2-[4,9-Dihydro-2,9-dimethyl-10,10-dioxo-2H-pyrazolo[3,4-c][2,1]benzothiazepin-4-yl)oxy] (examples 45 and 46.)

-2-[4,9-Dihydro-10,10-dioxo-2-ethyl-9-methyl-2H-pyrazolo[3,4-c][2,1]benzothiazepin-4-yl)oxy] (examples 47 and 48.)

-2-[2-Benzyl-4,9-dihydro-10,10-dioxo-9-methyl-2H-pyrazolo[3,4-c][2,1]benzothiazepin-4-yl)oxy] (examples 49 and 50.)

-2-[4,9-Dihydro-2,9-dimethyl-10,10-dioxothiazolo[5,4-c][2,1]benzothiazepin-4-yl)oxy] (examples 51 and 52.)

Example 53

5 ml. (0.04 mol) of a 33% solution of methylamine in ethanol are added to a solution of 1.94 g. (0.005 mol ) of the bromine derivative of example 33 in 15 ml. of tetrahydrofuran. The mixture is left at room temperature for 24 hours and then it is evaporated to dryness. The residue is dissolved in 4l. of toluene; it is washed with 3 x 25 ml. of water and the washed organic layer is extracted with 4 x 25 ml. of 20 % aqueous acetic acid solution. The acid extracts are joined, basified with a 20 % sodium hydroxide solution and extracted wotj ethyl acetate. After drying over anhydrous sodium sulfate, the organic phase is concentrated yielding a yellow oil that is dissolved again in 30 ml. of ethyl acetate. 0.58 g. (0.005 mol) of maleic acid in 5 ml. of ethanol are added to this solution, precipitating 2- [5,10-dihydro-4,4-dioxo-5-methylthieno[3,4-c] [2,1]-benzothiazepin-10-yl)oxy)-N-methylethylamine maleate as a white solid with a melting point of 172-175 ºC (ethanol).

I.R. (KBr): 1325, 1140 ($SO_2$)

Examples 54-108

Following the same process of example 53 the following derivatives of formula I ($R1 = CH_3$; $R_2 = H$; $R_3 = H$; $R_4 = O-(CH_2)_2-NR_5R_6$):

| Example nº | X | Y | Z | $R_5$ | $R_6$ |
|---|---|---|---|---|---|
| 54 | CH | CH | S | H | $CH_3$ |
| 55 | CH | CH | S | $CH_3$ | $CH_3$ |
| 57 | CH | CH | S | $(CH_2)_4$ | |
| 58 | CH | CH | S | $(CH_2)_5$ | |
| 59 | CH | CH | S | $(CH_2)_2$-NH-$(CH_2)_2$ | |
| 60 | CH | S | CH | $CH_3$ | $CH_3$ |
| 61 | CH | S | CH | $(CH_2)_4$ | |
| 62 | CH | S | CH | $(CH_2)_5$ | |
| 63 | CH | S | CH | $(CH_2)_2$-NH-$(CH_2)_2$ | |
| 64 | S | CH | CH | H | $CH_3$ |
| 65 | S | CH | CH | $CH_3$ | $CH_3$ |
| 66 | S | CH | CH | $(CH_2)_4$ | |
| 67 | S | CH | CH | $(CH_2)_5$ | |
| 68 | S | CH | CH | $(CH_2)_2$-NH-$(CH_2)_2$ | |
| 69 | $NCH_3$ | N | CH | H | $CH_3$ |
| 70 | $NCH_3$ | N | CH | $CH_3$ | $CH_3$ |
| 71 | $NCH_3$ | N | CH | $(CH_2)_4$ | |
| 72 | $NCH_3$ | N | CH | $(CH_2)_5$ | |
| 73 | $NCH_3$ | N | CH | $(CH_2)_2$-NH-$(CH_2)_2$ | |

| | | | | | |
|---|---|---|---|---|---|
| 74 | $NCH_2CH_3$ | N | CH | H | $CH_3$ |
| 75 | $NCH_2CH_3$ | N | CH | $CH_3$ | $CH_3$ |
| 76 | $NCH_2CH_3$ | N | CH | $(CH_2)_4$ | |
| 77 | $NCH_2CH_3$ | N | CH | $(CH_2)_5$ | |
| 78 | $NCH_2CH_3$ | N | CH | $(CH_2)_2$-NH-$(CH_2)_2$ | |
| 79 | $NCH_2C_6H_5$ | N | CH | H | $CH_3$ |
| 80 | $NCH_2C_6H_5$ | N | CH | $CH_3$ | $CH_3$ |
| 81 | $NCH_2C_6H_5$ | N | CH | $(CH_2)_4$ | |
| 82 | $NCH_2C_6H_5$ | N | CH | $(CH_2)_5$ | |
| 83 | $NCH_2C_6H_5$ | N | CH | $(CH_2)_2$-NH-$(CH_2)_2$ | |
| 84 | $NCH_3$ | N | $CCH_3$ | H | $CH_3$ |
| 85 | $NCH_3$ | N | $CCH_3$ | $CH_3$ | $CH_3$ |
| 86 | $NCH_3$ | N | $CCH_3$ | $(CH_2)_4$ | |
| 87 | $NCH_3$ | N | $CCH_3$ | $(CH_2)_5$ | |
| 88 | $NCH_3$ | N | $CCH_3$ | $(CH_2)_2$-NH-$(CH_2)_2$ | |
| 89 | N | $NCH_3$ | CH | H | $CH_3$ |
| 90 | N | $NCH_3$ | CH | $CH_3$ | $CH_3$ |
| 91 | N | $NCH_3$ | CH | $(CH_2)_4$ | |
| 92 | N | $NCH_3$ | CH | $(CH_2)_5$ | |
| 93 | N | $NCH_3$ | CH | $(CH_2)_2$-NH-$(CH_2)_2$ | |
| 94 | N | $NCH_2CH_3$ | CH | H | $CH_3$ |
| 95 | N | $NCH_2CH_3$ | CH | $CH_3$ | $CH_3$ |
| 96 | N | $NCH_2CH_3$ | CH | $(CH_2)_4$ | |
| 97 | N | $NCH_2CH_3$ | CH | $(CH_2)_5$ | |
| 98 | N | $NCH_2CH_3$ | CH | $(CH_2)_2$-NH-$(CH_2)_2$ | |
| 99 | N | $NCH_2C_6H_5$ | CH | H | $CH_3$ |
| 100 | N | $NCH_2C_6H_5$ | CH | $CH_3$ | $CH_3$ |
| 101 | N | $NCH_2C_6H_5$ | CH | $(CH_2)_4$ | |
| 102 | N | $NCH_2C_6H_5$ | CH | $(CH_2)_5$ | |
| 103 | N | $NCH_2C_6H_5$ | CH | $(CH_2)_2$-NH-$(CH_2)_2$ | |
| 104 | S | $CCH_3$ | N | H | $CH_3$ |
| 105 | S | $CCH_3$ | N | $CH_3$ | $CH_3$ |
| 106 | S | $CCH_3$ | N | $(CH_2)_4$ | |
| 107 | S | $CCH_3$ | N | $(CH_2)_5$ | |
| 108 | S | $CCH_3$ | N | $(CH_2)_2$-NH-$(CH_2)_2$ | |

Example 109

2.79 g. (0.01 mol) of the alcohol of example 9, 0.54 g. (0.002 mol ) of benzyltriethylammonium bromide and 1.70 g. (0.01 mol)of 1-(2-chloroethyl)pyrrolidine are added to a heterogeneous mixture, formed by 4 g. of sodium hydroxide, dissolved in 20 ml.of water and 50 ml. of toluene. Said mixture is refluxed, with good stirring for 10 hours, after which it is cooled; the organic phase is decanted and washed with 2 x 15 ml. of water. Once it is dry, the toluene solution is concentrated and yields a yellow oil that dissolves in 2-propanol and another solution of succinic acid in 2-propanol is added to it. In this way 2-[4,9-dihydro-2,9-dimethyl-10,10-dioxo-2H-pyrazolo[3,4-c][2,1]benzothiapin-4-yl)oxo]-1-ethylpyrrolidine succinate is precipitated as a white solid with a melting point of 130-133 $^{0}C$ ( ethanol) and whose analyticaland spectroscopic data coincide with those of example 91.

I.R.(KBr): 1330, 1160 ($SO_2$.)

Examples 110-163

By repeating the process described in example 109 the compounds of formula I whose symbols X, Y, Z, $R_1$, $R_2$, $R_3$, $R_4$, $R_5$, $R_6$ and $R_7$ coincide with those of examples 53-90 and 92-108, are obtained

Example 164

2.94 g. (0.01 mol) of the product obtained in example 20 are added to a solution of 0.46 g. (0.02 mol ) of sodium in 25 ml. of absolute ethanol , heated to reflux and the mixture is stirred at that temperature for 5 minutes. Afterwards, and maintaining the reflux, a solution of 1.70 g. (0.01 mol) of 1-(2-chloroethyl)-pyrrolidine hydrochloride in 15 ml. of ethanol begins to be dropped and stirred for another 3 hours, once all the amount to be added has been added. It is concentrated to dryness and the residue is extracted with 75 ml. of diethyl ether. After concentrating this last solution, a yellow oil is attained and it is dissolved again in absolute ethyl alcohol and saturated hydrogen chloride ether is added to it. A white solid is obtained and it has a melting point of 216-218 ºC (d) that turns out to be (Z)-5,10-dihydro-4,4-dioxo-10-(1-ethoxyiminopyrrolidine)-5-methyltheino[3,2-c][2,1]benzothiazepine.

I.R. (KBr): 1330, 1140 ($SO_2$.)

Examples 165-207

By repeating the process described in example 164, the desired compounds of formula I ($R_1 = CH_3$; $R_2 = H$; $R_3R_4 = N-O-(CH_2)_2-NR_5R_6$ are attained.

| Example nº | X | Y | Z | $R_5$ | $R_6$ |
|---|---|---|---|---|---|
| 165 | CH | CH | S | $CH_3$ | $CH_3$ |
| 166 | CH | CH | S | $(CH_2)_5$ | |
| 167 | CH | CH | S | $(CH_2)_2-O-(CH_2)_2$ | |
| 168 | CH | S | CH | $CH_3$ | $CH_3$ |
| 169 | CH | S | CH | $(CH_2)_4$ | |
| 170 | CH | S | CH | $(CH_2)_5$ | |
| 171 | CH | S | CH | $(CH_2)_2-O-(CH_2)_2$ | |
| 172 | S | CH | CH | $CH_3$ | $CH_3$ |
| 173 | S | CH | CH | $(CH_2)_4$ | |
| 174 | S | CH | CH | $(CH_2)_5$ | |
| 175 | S | CH | CH | $(CH_2)_2-O-(CH_2)_2$ | |
| 176 | $NCH_3$ | N | CH | $CH_3$ | $CH_3$ |

| | | | | | |
|---|---|---|---|---|---|
| 177 | $NCH_3$ | N | CH | $(CH_2)_4$ | |
| 178 | $NCH_3$ | N | CH | $(CH_2)_5$ | |
| 179 | $NCH_3$ | N | CH | $(CH_2)_2$-O-$(CH_2)_2$ | |
| 180 | $NCH_2CH_3$ | N | CH | $CH_3$ | $CH_3$ |
| 181 | $NCH_2CH_3$ | N | CH | $(CH_2)_4$ | |
| 182 | $NCH_2CH_3$ | N | CH | $(CH_2)_5$ | |
| 183 | $NCH_2CH_3$ | N | CH | $(CH_2)_2$-O-$(CH_2)_2$ | |
| 184 | $NCH_2C_6H_5$ | N | CH | $CH_3$ | $CH_3$ |
| 185 | $NCH_2C_6H_5$ | N | CH | $(CH_2)_4$ | |
| 186 | $NCH_2C_6H_5$ | N | CH | $(CH_2)_5$ | |
| 187 | $NCH_2C_6H_5$ | N | CH | $(CH_2)_2$-O-$(CH_2)_2$ | |
| 188 | $NCH_3$ | N | $CCH_3$ | $CH_3$ | $CH_3$ |
| 189 | $NCH_3$ | N | $CCH_3$ | $(CH_2)_4$ | |
| 190 | $NCH_3$ | N | $CCH_3$ | $(CH_2)_5$ | |
| 191 | $NCH_3$ | N | $CCH_3$ | $(CH_2)_2$-O-$(CH_2)_2$ | |
| 192 | N | $NCH_3$ | CH | $CH_3$ | $CH_3$ |
| 193 | N | $NCH_3$ | CH | $(CH_2)_4$ | |
| 194 | N | $NCH_3$ | CH | $(CH_2)_5$ | |
| 195 | N | $NCH_3$ | CH | $(CH_2)_2$-O-$(CH_2)_2$ | |
| 196 | N | $NCH_2CH_3$ | CH | $CH_3$ | $CH_3$ |
| 197 | N | $NCH_2CH_3$ | CH | $(CH_2)_4$ | |
| 198 | N | $NCH_2CH_3$ | CH | $(CH_2)_5$ | |
| 199 | N | $NCH_2CH_3$ | CH | $(CH_2)_2$-O-$(CH_2)_2$ | |
| 200 | N | $NCH_2C_6H_5$ | CH | $CH_3$ | $CH_3$ |
| 201 | N | $NCH_2C_6H_5$ | CH | $(CH_2)_4$ | |
| 202 | N | $NCH_2C_6H_5$ | CH | $(CH_2)_5$ | |
| 203 | N | $NCH_2C_6H_5$ | CH | $(CH_2)_2$-O-$(CH_2)_2$ | |

| | | | | | |
|---|---|---|---|---|---|
| 204 | S | $CCH_3$ | N | $CH_3$ | $CH_3$ |
| 205 | S | $CCH_3$ | N | $(CH_2)_4$ | |
| 206 | S | $CCH_3$ | N | $(CH_2)_5$ | |
| 207 | S | $CCH_3$ | N | $(CH_2)_2$-O-$(CH_2)_2$ | |

Example 208

1.46 g. (0.005 mol ) of (Z)-4,9-dihydro-2,9-dimethyl-10,10-dioxo-4-hydroxyimino-2H-pyrazolo[3,4-c][2,1]-benzothiazepine, along with 0.87 g. (0.006 mol ) of 1-chloro-2-dimethylaminoethane hydrochloride and 0.27 g. (0.001 mol) of benzyltriethylammonium bromide are added to a mixture of 2 g. of sodium hydroxide dissolved in 10 ml. of water and 25 ml. of toluene.
Said mixture is refluxed, with good stirring, for 15 hours and, after cooling, the aqueous phase is decanted. The organic phase is washed with 3 x 15 ml. of water and is dried over anhydrous sodium sulfate. Once it is dry, the toluene is concentrated at reduced pressure, yielding the corresponding oxime ether as a yellow oil that is transformed into maleate with a melting point of 176-179 $^{o}$C (d).

I.R. (KBr): 1340, 1170 ($SO_2$ ).

Examples 209-253

By means of substituting in example 208 the hydroxyimino derivative and the chloroaminoethane by suitable reagents the compounds of formula I described in examples 164-207 are obtained.

Example 254

0.58 g. (0.024 mol ) of magnesium turnings are covered with 10 ml. of dry tetrahydrofuran in a stream of nitrogen. An iodine crystal is added to activate the magnesium and subsequently a solution of 3.5 g. (0.028 mol ) of 3-dimethylamino-1-propyl chloride in 6 ml. of dry tetrahydrofuran. Said mixture is heated to reflux for 1 hour, it is cooled to room temperature and a solution of 4.74 g. (0.017 mol) of 5,10-dihydro-5-methyl-4,4,10-trioxothieno[3,2-c][2,1]benzothiazepine in 50 ml. of dry tetrahydro furan at 60º C is dropped on to it. Once all of the ketone has been added, the reaction is stirred for one more hour at room temperature and it is poured on to 50 ml. of 10% aqueous ammonium chloride. It is stirred and the obtained solid is filtered, washed with water and dried, yielding a white solid with a melting point of 186-188 ºC (ethanol.)

I.R. (KBr): 2600 (OH); 1310, 1150 ($SO_2$.)

Examples 255-265

In a manner similar to that used in example 254 the following compounds are synthesized:

-5,10-Dihydro-10-(3-dimethylamino-1-propyl)-4,4-dioxo-10-hydroxy-5-methylthieno[3,4-c][2,1]-benzothiazepine (example 255.)

-4,9-Dihydro-4-(3-dimethylamino-1-propyl)-10,10-dioxo-4-hydroxy-9-methylthieno[2,3-c][2,1]-benzothiazepine (example 256.)

-4,9-Dihydro-4-(3-dimethylamino-1-propyl)-10,10-dioxo-4-hydroxy-9-methyl-1H-pyrazolo[3,4-c][2,1]-benzothiazepine (example 257.)

-4,9-Dihydro-1,9-dimethyl-4-(3-dimethylamino-1-propyl)-10,10-dioxo-4-hydroxy-1H-pyrazolo[3,4-c][2,1]-benzothiazepine (example 258.)

-4,9-Dihydro-4-(3-dimethylamino-1-propyl)-10,10-dioxo-1-ethyl-4-hydroxy-9-methyl-1H-pyrazolo[3,4-c]-[2,1]benzothiazepine (example 259.)

-1-Benzyl-4,9-dihydro-4-(3-dimethylamino-1-propyl)-10,10-dioxo-4-hydroxy-9-methyl-1H-pyrazolo[3,4-c]-[2,1]benzothiazepine (example 260.)

-4,9-Dihydro-4-(3-dimethylamino-1-propyl)-10,10-dioxo-4-hydroxy-1,3,9-trimethyl-1H-pyrazolo[3,4-c][2,1]-benzothiazepine (example 261.)

-4,9-Dihydro-2,9-dimethyl-4-(3-dimethylamino-1-propyl)-10,10-dioxo-4-hydroxy-2H-pyrazolo[3,4-c][2,1]-ben zothiazepine (example 262.)

-4,9-Dihydro-4-(3-dimethylamino-1-propyl)-10,10-dioxo-2-ethyl-4-hydroxy-9-methyl-2H-pyrazolo[3,4-c]-[2,1]benzothiazepine (example 263.)

-2-Benzyl-4,9-dihydro-4-(3-dimethylamino-1-propyl)-10,10-dioxo-4-hydroxy-9-methyl-9-methyl-2H-pyrazolo[3,4-c][2,1]benzothiazepine (example 264.)

-4,9-Dihydro-2,9-dimethyl-4-(3-dimethylamino-1-propyl)-10,10-dioxo-4-hydroxythiazolo[5,4-c][2,1]-benzothiazepine (example 265.)

Example 266

1.46 g. (0.004 mol) of thieno[2,1]benzothiazepine of example 255 are added in portions to 8 ml. of concentrated sulfuric acid. It is stirred at room temperature for half an hour and 10 ml. of ice water are added to it. It is basified with 20% aqueous sodium hydroxide and extracted with 4 x 15 ml. of ethyl ehter. The organic diethyl extracts are collected, washedwith 10 ml. of brine and drived over anhydrous sodium sulfate. After concentrating to dryness a white solid that turns out to be the mixture of Z and E isomers (8% (Z) and 92% (E)), of 5,10-dihydro-4-(3-dimethylamino-1-propyliden)-4-4-dioxo-5-methyltheino[3,4-c][2,1]-benzothiazepine. Subsequently said mixture is separated by fractionated crystallization and chromatography.

Examples 267-277

By using a similar process the 3-dimethylamino-1-propyliden derivatives of examples 254 and 256-265 are obtained.

Pharmacological use

The derivatives of formula I are basic compounds, due to the presence of an aminic nitrogen and they form stable salts with several acids. Biologically acceptable acid salts are preferred (J. Pharm. Sci. 66, 1 (1977)); among the inorganic acids are hydrochloric, hydrobromic , sulfuric, nitric and phosphoric acids, while some examples of the organic acids include acetic, citric, fumaric, maleic, succinic and tartaric acids. The salts of the compounds of formula I with these acids can be easily formed by any of the conventional methods. The above mentioned acids are given only as examples and they do not limit at all the possibilities of salification of the invention.

The compounds of formula I are antidepressant, diuretic, antihypertensive, antihistamic, anticonvulsive, antipyretic, anti-inflammatory and/or analgesic agents. For example, 5,10-dihydro-4,4-dioxo-10-hydroxy-5-methylthieno[3,2-c][2,1]benzothiazepine (example 1) shows a 92% inhibition of pain at 100 mg/kg p.o. in the Siegmund test [Proc. Soc. Exp. Biol. Med. 95, 729 (1957.)] In connection with its activity, this compound is very safe with a $LD_{50}$ of 1250 mg/kg p.o. in mice.

The antidepressant activity of these compounds is measured in terms of their capacity of antagonizing the effects of tetrabenazine (ptosis, akinesia and hypothermia), this action being characteristic of antidepressants such as amitriptiline or thianeptine (New Drugs: Discovery and Development. A.A. Rubin (Ed.), Marcel Dekker, Inc. New York-Basel, 203 (1978.)) For example, 2-[(5,10-dihydro-4,4-dioxo-5-methylthieno[3,4-c]-[2,1]benzothiazepin-10-yl)oxy]-N-methylethylamine (example 53) and 2-[(4,9-dihydro-2,9-dimethyl-10,10-dioxo-2H-pyrazolo[3,4-c][2,1]-benzothiazepin-4-yl)oxy]-1-ethylpyrrolidine (example 91) prevents ptosis at 25 mg/kg p.o., 100% and 90%, respectively, while the antagonism to hypothermia produced by tetrabenazine when administered is 85 % and 75 %, respectively.

The compounds of formula I and their pharmacologically acceptable salts are active orally and parenterally. These substances can be administered in conventional pharmaceutical forms, included within the scope of the present invention, which comprise tablets and capsules for oral administration and suspensions and solutions for oral or parenteral administration.

**Claims**

**1.** Pharmacologically active tricyclic derivatives of general formula:

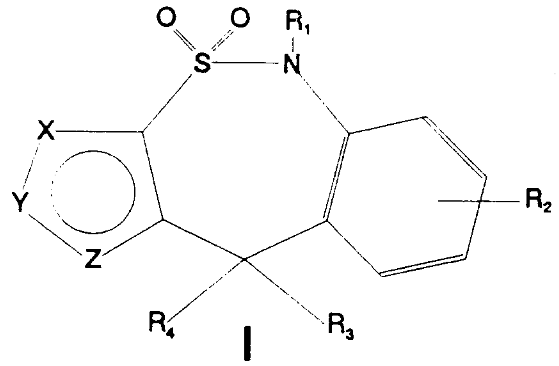

I

wherein X represents a nitrogen atom, a sulfur atom or a NH,N-alkyl, N-arylalkyl or CH group; Y represents a nitrogen atom, a sulfur atom or a NH,N-alkyl, N-arylalkyl, CH or C-alkyl group and Z is a nitrogen atom, a sulfur atom or a CH, C-alkyl or C-aryl group; $R_1$ represents a hydrogen atom, linear or branched alkyl groups, or arylalkyl groups wherein there may be functional substitutions in their nucleus or branches in their chain; $R_2$ indicates one or several substituents bonded to the benzene portion of the structure, selected from among hydrogen atoms, halogen atoms and/or nitro, amino, lower-alkyl amino, lower-dialkyl amino, cyano, sulfonamide, trifluoromethyl and alkyl groups or alkoxy with 1 to 6 carbon atoms; $R_3$ and $R_4$, identical or different, represent independently from one another, a hydrogen atom, a halogen atom, hydroxy, lower-alkoxy, mercapto, lower-alkyl, amino, lower-alkyl amino, lower-dialkyl amino groups or a group of formula III:

T-$(Alk)_n$-Het III

wherein T represents an oxygen atom, a nitrogen atom or a sulfur atom; n is equal to 0 or 1; Alk represents a linear or branched chain of 1 to 5 carbon atoms; Het represents a mono or bicyclic saturated heterocyclic system with 5 to 7 vertexes in each cycle optionally including 1 or 2 heteroatoms selected from among nitrogen, oxygen and sulfur with the condition that the heterocyclic system contains at least one nitrogen atom, each nitorgen atom being optionally substituted by a lower-alkyl group, a phenyl group, a phenyl lower alkyl group, the phenly nucleus likewise optionally substituted by one or several halogen atoms or lower-alkyl, lower alkoxy or trifluoromethyl groups; Het may also be represented by a group of formula

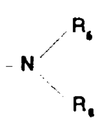

wherein $R_5$ and $R_6$, identical or different, represent independently from each other a hydrogen atom, a lower-alkyl group or a lower aryalkyl group; $R_3$ and $R_4$ can be bonded forming an imino group, a hydroxyimino group, a lower-alkoxy imino group or a group of formula

= N-$(Alk)_n$-Het IVa

or a group of formula

= N-O-$(Alk)_n$-Het IVb

or a group of formula

= $(Alk)_n$-Het IVc

or a group of formula

= Het IVd

wherein Het, Alk and n correspond with the above mentioned meaning, it being understood that lower-alkyl, lower-alkoxy and lower alkylthio groups are understood as linear or branched groups containing from 1 to 6 carbon atoms, their pharmacologically acceptable salts and their isomers, enantiomers, diastereoisomers, either isolated or in mixtures thereof.

**2.** Compounds according to claim 1, derived from the 5,10-dihydro-4,4-dioxothieno[3,2-c][2,1]-benzothiazepine system and isomers, enantiomers and diastereoiosomers thereof, either isolated or in mixtures thereof, as well as pharmaceutically acceptable acid addition salts thereof.

**3.** Compounds according to claim 1, derived from the 5,10-dihydro-4,4-dioxothieno[3,4-c][2,1]-benzothiazepine system and isomers, enantiomers and diastereoisomers thereof, either isolated or in mixtures thereof, as well as pharmaceutically aacceptable acid addition salts thereof.

**4.** Compounds according to claim 1, derived from the 4,9-dihydro-10,10-dioxothieno[2,3][2,1]-benzothiazepine system and isomers, enantiomers and diastereoisomers thereof, either isolated or in minxtures thereof, as well as pharmaceutically acceptable acid addition salts thereof.

**5.** Compounds according to claim 1, derived from the 4,9-dihydro-10,10-dioxo-1H-pyrazolo[3,4-][2,1]-benzothiazepine system and isomers, enanotiomers and diastereoisomers thereof, either isolated or in mixtures thereof, as well as pharmaceutically acceptable acid addition salts thereof.

**6.** Compounds according to claim 1, derived from the 4,9-dihydro-10,10-dioxo-2H-pyrazolo[3,4-c][2,1] system and isomers, enantiomers and diastereoisomers thereof, either isolated or in mixtures thereof, as well as pharmaceutically acceptable acid addition salts thereof.

**7.** Compounds according to claim 1, derived from the 4,9-dihydro-10,10-dioxothiazolo[5,4-c][2,1]-benzothiazepine system, and isomers, enantiomers and diastereoisomers thereof, either isolated or in mixtures thereof, as well as acid addition salts thereof.

**8.** Compounds according to claim 1, which is 5,10-dihydro-4,4-dioxo-10-hydroxy-5-methylthieno[3,2-c][2,1]-benzothiazepine and isomers, enantiomers and diastereoisomers thereof, either isolated or in mixtures thereof.

[(5,10-dihydro-4,4-dioxo-5-methylthieno[3,4-c][2,1]benzothiazepin-10-yl)oxy]N-methylethylamine and isomers, enantiomers and diastereoisomers thereof, either isolated or in mixtures thereof.

**10.** Compounds according to claim 1 which is 2-[4,9-dihydro-2,9-dimethyl-10,10-dioxo-2H-pyrazolo[3,4-c]-[2,1]benzothiazepin-4-yl)oxy]1-ethylpyrrolidine and isomers, enantiomers and diastereoisomers thereof, either isolated or in mixtures thereof.

**11.** Process to obtain compounds of general formula I ($R_3$;H and $R_4$:O-Alk-$NR_5R_6$;$R_3$:H and $R_4$:S-Alk-$NR_5R_6$;$R_3$– :H and $R_4$:HN-Alk-$NR_5R_6$), according to claim 1, which comprises the reaction between an alcohol, a thiol or a tricyclic amine of formulae VII, VIII and IX, respectively, and an aminoalkylic derivative having a W reactive group, in an organic solvent and in the presence of a base, organic or inorganic, or else in a heterogeneous mixture comprised of a water immiscible organic solvent and an aqueous solution of a base, preferably an alkali metal hydroxide, all in the presence of a quaternary ammonium salt or a phosphonium salt, according to the following diagram

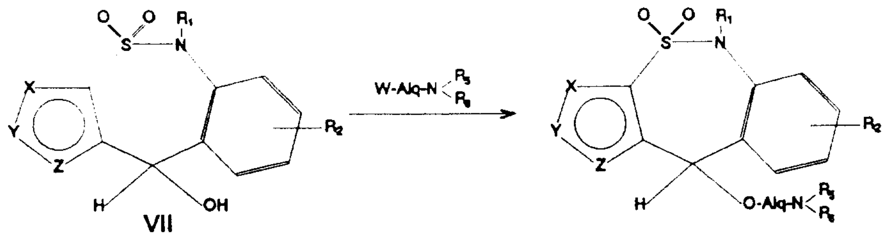


VII

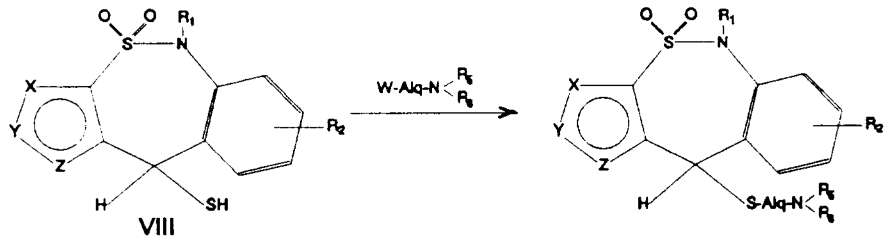


VIII

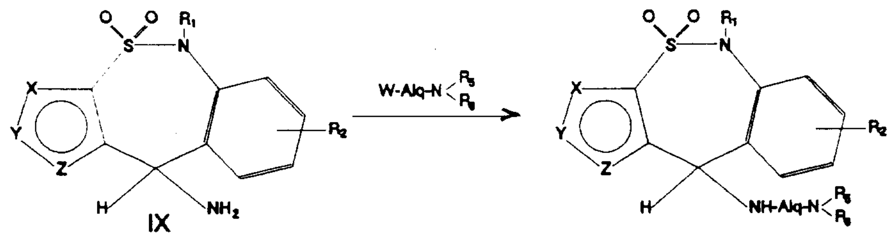


IX

wherein the W reactive group represents chlorine, bromine, iodine, alkylsulfate, alkylsulfonate, arylsulfonate or alkylphosphate and the rest of the symbols are defined as in claim 1.

**12.** Process to obtain compounds of general formula I ($R_3$:H and $R_4$:O-Alk-$NR_5R_6$; $R_3$:H and $R_4$:S-Alk-$NR_5R_6$;R :H and $R_4$HN-Alk-$NR_5R_6$), according to claim 1, which comprises the reaction between a tricyclic derivative of formula X and hydroxyalkamines, mercaptoalkylamines and aminoalkylamines, or salts thereof, formed by reacting with strong bases, in an organic solvent, or a heterogeneous mixture comprised of a water immiscible organic solvent and an aqueous solution of a base, preferably an alkali metal hydroxide, all in the presence of a quaternary ammonium salt, or a phosphonium salt, according to the following diagram

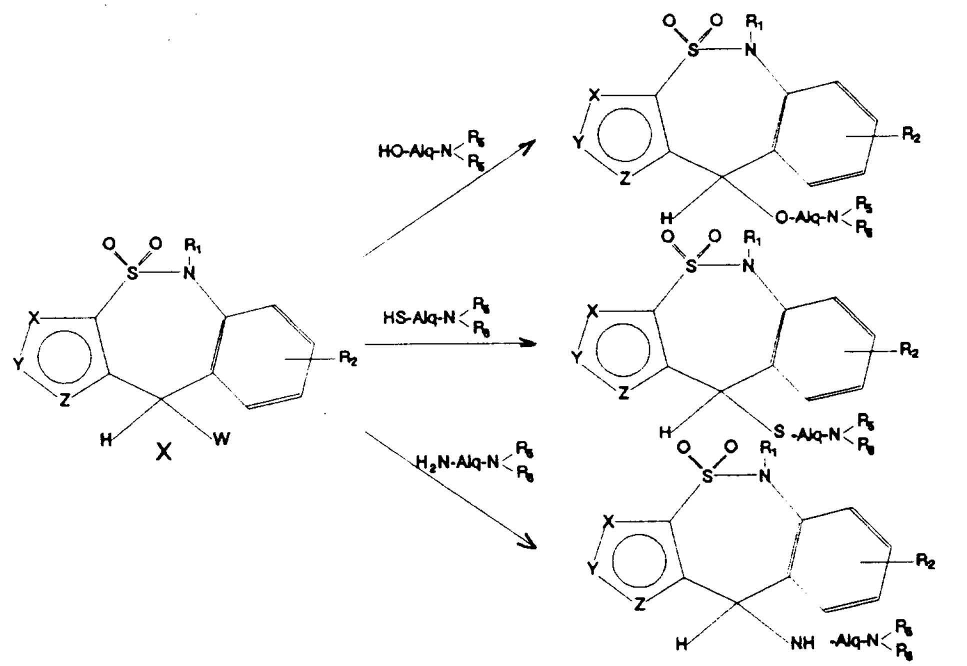


X

wherein all the symbols are defined as in claims 1 and 8.

**13.** Process to obtain compounds of general formula I ($R_3$:H and $R_4$:O-Alk-$NR_5R_6$), according to claim 1, which comprises the successive carrying out of the following two reaction steps:

a) Dehydrating a tricyclic alcohol of formula VII with an aliphatic alcohol having a W reactive group, separated by, at least, two carbon atoms from the hydroxyl group, to form a derivative of formula XI according to the following diagram:

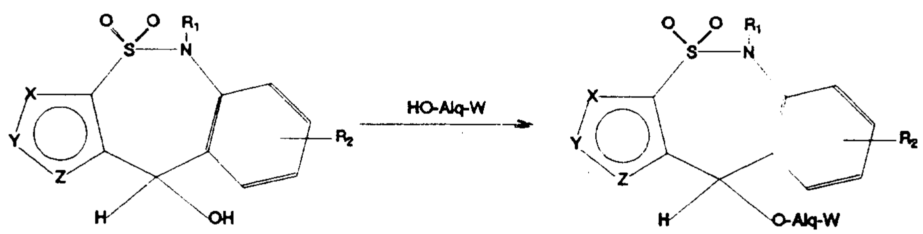


VII XI

b) Substituting the W group by amines of formula $HNR_5R_6$, according to the diagram:

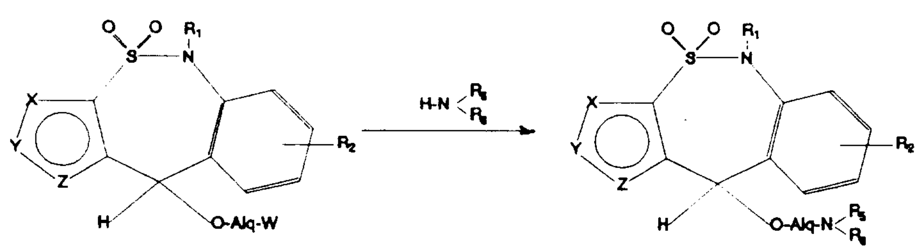


XI

The first step is carried out in an inert organic solvent and in the presence of a dehydrating agent, preferably p-toluenosulfonic acid. The second step is carried out in an organic solvent and a base, preferably an excess of the amine used. The symbols of the different formulae are defined as in claims 1 and 8.

**14.** Process to obtain compounds of general formula I ($R_3R_4$:N-O-Alk-$NR_5R_6$), according to claim 1, which comprises the reaction between an oxime salt of formula XIII, preferably an alkaline metal and a conveniently substituted aminoalkyl derivative, such as is represented in the following diagram:

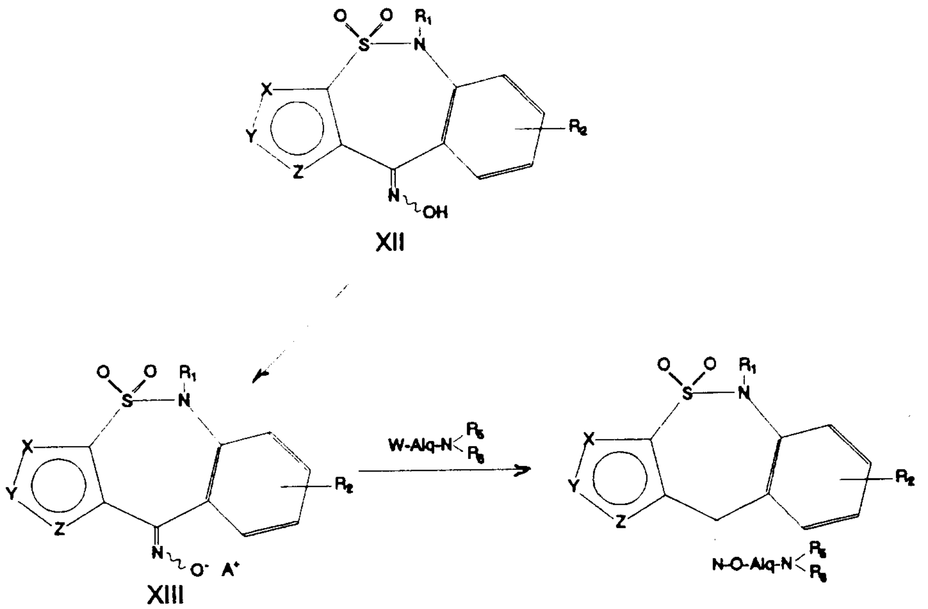


XII

XIII

In this diagram the meanings of all the symbols are defined as in claim 1, and this implies, either treating the oxime in an anhydrous medium with a strong base, such as an alkaline metal alkoxide, preferably sodium ethoxide, an alkali metal or alkaline-earth metal, or a lithium alkyl, or treating the oxime in a heterogeneous basic medium and in the presence of a phase transfer catalyst, preferably an ammonium salt, a phosphonium salt or a crown ether.

**15.** Process to obtain compounds of general formula I ($R_3R_4$:N-O-Alk-$NR_5R_6$), according to claim 1, which comprises the reaction between a hydroxyalkylamine and a derivative of formula XIV, according to the following diagram:

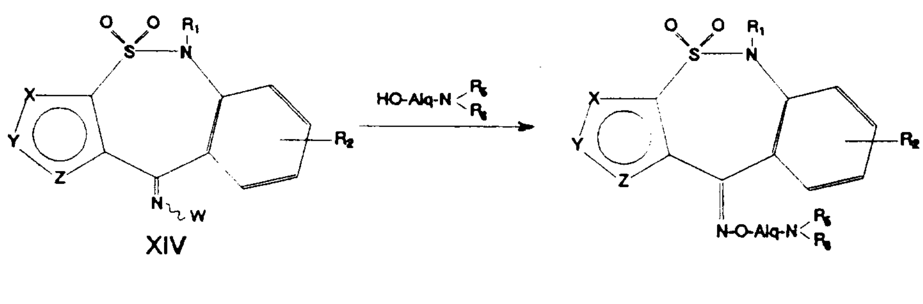


XIV

in which case the W reactive group has the meaning of alkylsulfonate, arylsulfonate or alkylphosphate and the rest of the symbols are defined as in claims 1 and 11.

**16.** Process to obtain compounds of general formula I ($R_3R_4$:N-O-Alk-$NR_5R_6$), according to claim 1, which comprises condensing a ketone of formula V with conveniently substituted hydroxylamines, in an organic solvent, as is shown in the following diagram.

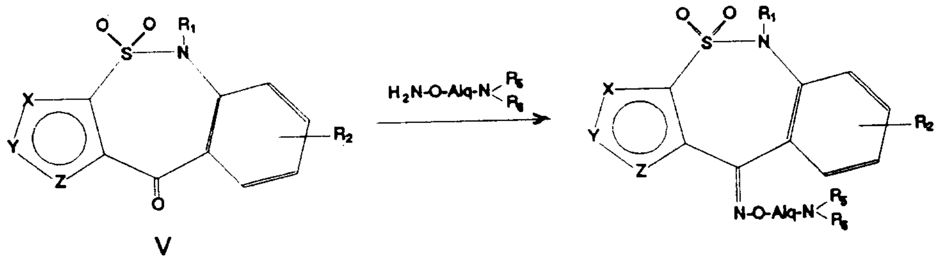


V

The symbols X, Y, Z, $R_1$, $R_2$, Alk, $R_5$ and $R_6$ are defined as in claims 1 and 11.

**17.** Process to obtain compounds of general formula I ($R_3R_4$:N-O-Alk-$NR_5R_6$), according to claim 1, which comprises carrying out successively the following two reaction steps:

a) Condensing a ketone of formula V with a Grignard reagent of an aminoalkyl halide or of a nitrogenated heteocyclic halide, preferably a chloride, bromide or iodide, in an inert organic solvent, selected from among aliphatic or aromatic hydrocarbons or ethers, preferably tetrahydrofuran and subsequent hydrolysis of the complex with an aqueous solution, preferably ammonium chloride.

b) Dehydrating the above hydroxylic derivatives, by treatment with strong, organic or inorganic, acids, preferably sulfuric acid, or else with organic dehydrating agents, preferably acetic anhydride, or inorganic dehydrating agents, selected from among phosphorus oxychloride, thionyl chloride, sulfuryl chloride, phosphorus pentachloride, etc. according to the following diagram:

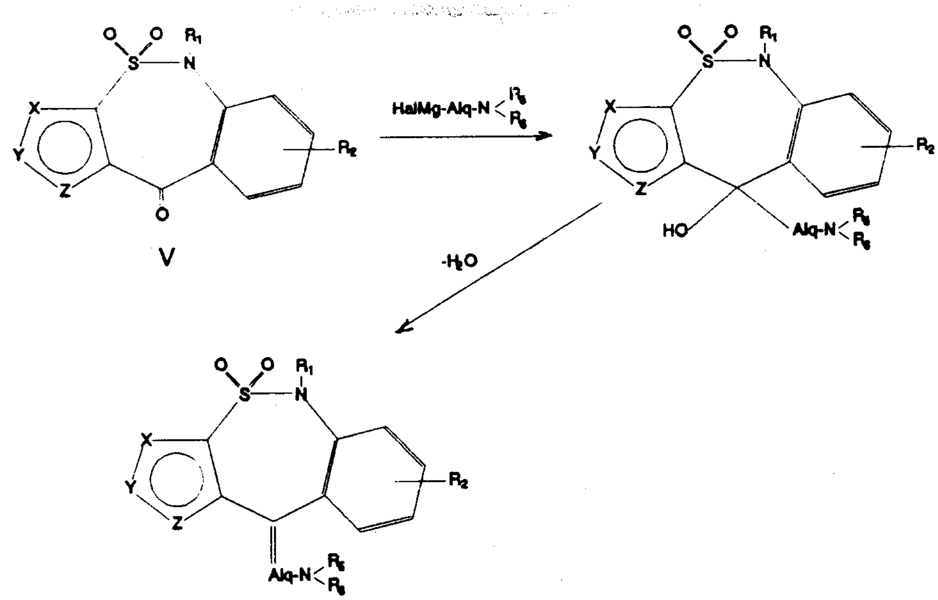


wherein the symbol Hal represents chlorine, bromine or iodine and the rest of the symbols are defined as in claim 1.

**18.** Pharmaceutical compositons that comprise, as the active ingredient, at least one of the compounds claimed in any of claims 1 to 7, or a pharmacologically acceptable salt thereof, in association with a pharmaceutically acceptable vehicle or diluent.

**19.** Pharmaceutical compositions according to claim 15, useful to treat psychotropic disorders, arterial hypertension, diuresis, convulsions, allergies, febrile, painful and inflammatory processes.

European Patent Office

# EUROPEAN SEARCH REPORT

Application Number

EP 92 20 3913

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,P,A<br><br><br><br><br><br><br>D,A | CHEMICAL ABSTRACTS, vol. 116, no. 21, 25 May 1992, Columbus, Ohio, US; abstract no. 214543s, V. NOVEROLA ET AL. 'Preparation of new trioxothienobenzothiazepines' page 752 ; * abstract *<br>& ES-A-2 021 548<br>--- | 1,19 | C07D513/04<br>A61K31/55<br>//(C07D513/04, 281:00,231:00)<br>(C07D513/04, 333:00,281:00)<br>(C07D513/04, 281:00,277:00) |
| A | EP-A-0 239 436 (ADIR ET COMPANIE)<br>* claims 1,3,5 *<br>----- | 1,19 | |
| | | | TECHNICAL FIELDS SEARCHED (Int. Cl.5 ) |
| | | | C07D<br>A61K |

The present search report has been drawn up for all claims

| Place of search | Date of completion of the search | Examiner |
|---|---|---|
| THE HAGUE | 17 MARCH 1993 | VOYIAZOGLOU D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)